# EUROPEAN PATENT APPLICATION

(11) **EP 1 352 977 A2**
(43) Date of publication of application: **15.10.2003**
(21) Application number: 03447089.8
(22) Date of filing: 10.04.2003
(51) Int. Cl.: C12R 1/00, C12N 9/14

(54) **Novel Desulfitobacterium strain for the degradation of chloroalkenes**

(30) Priority: 10.04.2002 EP 02447061
(71) Applicant: UNIVERSITEIT GENT, 9000 Gent (BE)
(72) Inventor: De Wildeman, Stefaan, 3010 Kessel-lo (BE); Verstraete, Willy, 9032 Wondelgem (BE)
(74) Representative: Bird, William Edward

(57) **Abstract**

The present invention relates to a novel species of the *Desulfitobacterium* genus capable of dehalogenating haloalkanes under anaerobic conditions. The invention relates further to compositions and methods for dehalogenating haloalkanes. The properties of the novel bacterium of the present invention allows applications such as the in situ remediation of anaerobic contaminated sites. Halogenated compounds which can be degraded with the bacteria of the present invention include pollutants such as 1,2-dichloroethane and 1,2-dichloropropane.

## Description

### FIELD OF THE INVENTION

The invention relates to novel bacteria, methods and apparatus for the microbial degradation of halogenated alkanes. The invention has applications such as the in situ remediation of sites contaminated with haloalkanes such as chloroalkanes.

### BACKGROUND

Prevention of groundwater pollution, especially by halogenated, and more in particular by chlorinated solvents is a worldwide problem associated primarily with industrial sites where mishandling or improper disposal has brought these solvents in contact with the soil. Of all toxic pollutants that have been disposed to groundwater and soil, several chlorinated solvents occupy the top rankings of the American and European priority lists. Most of them are hazardous compounds for humans and wildlife. Moreover, several of them are suspected carcinogens (class II), while others have been confirmed to be carcinogenic (class I). Examples of well-known molecules belonging to classes II and I are respectively 1,2-dichloroethane (1,2-DCA) and vinylchloride (VC). Both are intermediate compounds in the production of PVC. In addition, chlorinated solvents are soluble in groundwater and can therefore be transported to drinking water reservoirs where they may pose serious health hazards. Therefore, it is of vital importance from the viewpoint of environmental protection to treat contaminated aqueous media such as solvent refuse, underground water and soil by removing and degrading the halogenated organic compounds contained therein.

Conventional methods used to remediate chlorinated solvents include the removal of polluted ground water and subsequent treatment in industrial plants, vacuum extraction, and site excavation. These intensive technologies have high or even prohibitive costs when used to treat large sites. In addition, the method requires extended time periods for treatment and in some instances creates additional waste. Use of processes that stimulate *in situ* degradation of contaminants by microbes or other microorganisms, called "bioremediation", can reduce the substantial expense typically associated with contaminated groundwater cleanup. This biological approach may utilize microorganisms indigenous to a particular site where the remediation process consists primarily of making additions to the contaminated site that enhance the growth of the desired microorganism. Alternatively, non-indigenous microorganisms may be introduced to a contaminated site with the necessary amendments needed for growth.

Aerobic approaches to *in situ* bioremediation may be applied. However, converting groundwater for instance into an oxidizing environment is difficult and expensive, largely due to the high cost and engineering challenge associated with the subsurface delivery of oxygen. Input of oxygen can cause groundwater disruption and silt up of the underground matrix.

Anaerobic or substantially anaerobic approaches to *in situ* bioremediation are less expensive and less invasive than aerobic approaches. In anaerobic environments, chlorinated solvents can be bioremediated in a process of sequential chloride removal called "reductive dechlorination". In this process, the microorganisms use the chlorinated solvent as an electron acceptor, while using either a reduced carbon compound or hydrogen as an electron donor. As a consequence of the reductive dechlorination process non-hazardous chloride anions are released.

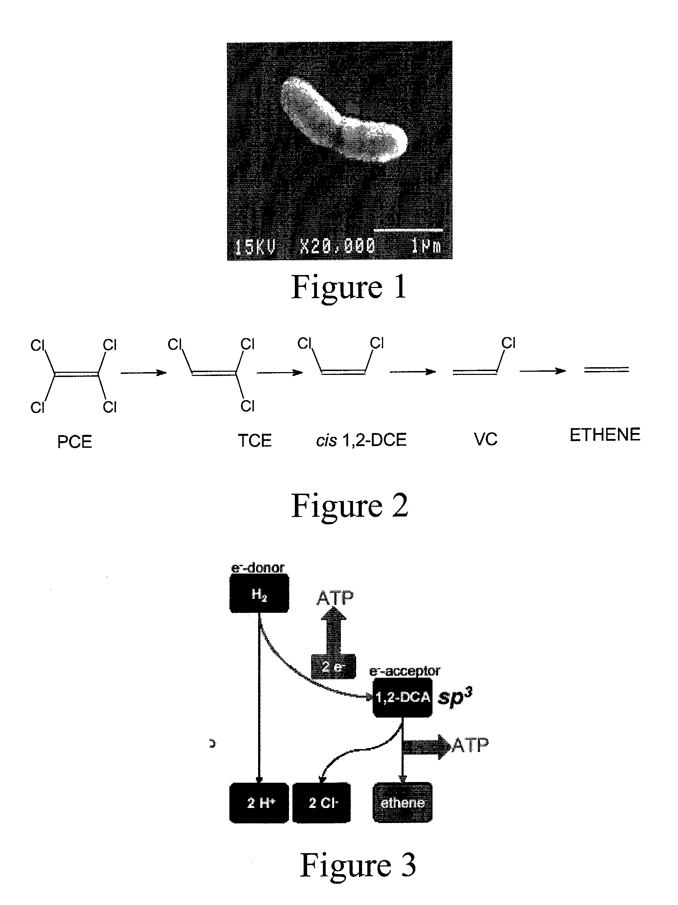

A number of microorganisms are known to catalyze the anaerobic conversion of persistent chlorinated hydrocarbons into lower chlorinated compounds. When hydrogen or formate are used as the direct electron donor for these dechlorinations, these microorganisms are referred to as dehalorespiring organisms. The latter means that they gain energy from respiring the chlorinated hydrocarbons. Many details have been elucidated concerning the anaerobic microbial dechlorination of the chlorinated ethenes perchloroethene (PCE) and trichloroethene (TCE), both toxic dry-cleaning agents. The conversion steps of PCE for instance are represented in figure 2 *Dehalospirillium multivorans* has been shown to partially de-chlorinate chlorinated ethenes and can respire PCE to *cis*-1,2-DCE (Sholz-Maramatsu et al. 1995, Arch.

Microbiol. 163:48-56). Also microorganisms belonging to the genus *Dehalococcoides* were shown to be dehalorespiring microorganisms. For instance, *Dehalococcoides ethenogenes* strain 195 (Maymo-Gatell et al. 1997, Science 276:1568-1571), is able to gain energy from the reduction of 1,2-DCE to VC (Maymo-Gatell et al. 2001, Environ. Sci. Technol. 65:516-521). VC itself is then further reduced to ethene at very low conversion rates. The TCE-dehalogenase enzyme of *Dehalococcoides ethenogenes* strain 195 performs the reduction from TCE to ethene in three subsequent reductive hydrogenolysis reactions. *Dehalococcoides* strain CBDB1 can respire some tri- and tetrachlorobenzenes (Adrian et al. 2000, Nature 408:580-583) producing dichlorobenzenes. Another important genus, respiring PCE, TCE and an extended range of chlorophenols is the *Desulfitobacterium* genus.

*Dehalococcoides ethenogenes* strain 195 has been described to be active under anaerobic conditions and to degrade 1,2-DCA into non-chlorinated compounds (Mayma-Gatell et al. 1999, Appl. Environ. Microbiol. 65:3108-3133). However, this bacterial strain 195 can not be effectively used in commercial industrial applications for *in situ* remediation of soils and groundwater contaminated with chlorinated alkanes. The oxygen sensitivity and small metabolic substrate spectrum of this *Dehalococcoides ethenogenes* strain 195 are important barriers for mass production and controlled applications. In addition, the molar selectivity for 1,2-DCA conversion to ethene by this micro-organism comprises only 99%, which means that still a considerable amount of 1% of the toxic compound VC is produced during the dehalorespiration of 1,2-DCA.

Besides this, chloroethanes were shown to be partly dechlorinated at slow rates in co-metabolic conversions of methanogens and acetogens. However, application of the latter bacteria for cleaning up of chloroalkane contaminated aquifers is not feasible because of the incompleteness and slow conversion speed of the dechlorination.

The key-players involved in the bacterial degradation of 1,2-dichloropropane (1,2-D) are yet unknown. No anaerobic 1,2-D dehalorespiring bacterium has been isolated so far.

Several patent applications relate to the anaerobic remediation of groundwater or soil contaminated with chlorinated alkenes and alkanes by using microorganisms. For instance, the patent application EP-A2-0864542 describes a method for degradation of chlorinated ethenes and propenes in groundwater, whereby the groundwater is mixed to an electron donor and conducted through a reactor, which contains a bacterial consortium able to degrade the chlorinated alkenes under anaerobic conditions. US patent No 6,001,252 and US patent No 2002/0015991 A1 both describe a method for anaerobic *in situ* remediation of groundwater from chlorinated alkenes or alkanes. The method comprises inoculating the site with a microbial consortium capable of dechlorination under substantially anaerobic conditions. However, none of the above-mentioned patent applications describes the *in situ* remediation of the most important and most hazardous di- and trichloroalkanes found in groundwater or soil including 1,2-dichloroethane (1,2-DCA), 1,2-dichloropropane (1,2-D), 1,1,2-trichloroethane (1,1,2-TCA), 2,3-dichlorobutane (2,3-DCB) or 1,2-dichlorobutane (1,2-DCB).

*Ex situ* destruction of *C*2 to *C*4 chloroalkanes has been achieved with aerobic biomass, containing bacteria that utilize some of these compounds as carbon and energy source (Janssen *et al.* 1985). In bioreactors containing membranes or activated carbon, convincing results were obtained in the aerobic bioremediation of 1,2-DCA containing groundwater (Dossantos and Livingston 1993a; Dossantos and Livingston 1993b; Dossantos and Livingston 1994; Stucki and Thuer 1994; Dossantos and Livingston 1995; Stucki and Thuer 1995). Reactors were reported wherein the microbial degradation under anaerobic conditions of compounds such as 1,2 -DCA are described (Wild et al.. (1995) *Biodegradation* **6,** 309-318.). This method still requires the transport of the substrate to the reactor, a need for permanent anaerobic conditions and has a low efficiency.

There remains a long felt need in the art for an oxygen-tolerant and easy to grow bacterial strain which can completely and rapidly degrade toxic and hazardous haloalkanes and especially di- and trichloroalkanes such as 1,2-dichloroethane (1,2-DCA), 1,2-dichloropropane (1,2-D), 1,1,2-trichloroethane (1,1,2-TCA), 2,3-dichlorobutane (2,3-DCB) and 1,2-dichlorobutane (1,2-DCB). In addition, there is also a great need for an inexpensive rapid, effective and environmentally friendly method for the *in situ* remediation of soils and groundwater contaminated with the important above-mentioned chlorinated alkanes.

### SUMMARY OF THE INVENTION

The present invention provides a novel bacterial strain capable of rapid dehalogenation of haloalkanes and especially dechlorinating di- and trihaloalkanes. Surprisingly high conversion rates can be obtained. The present invention also provides an inexpensive, very effective and rapid method for the complete *in situ* bioremediation of halogenated, especially chlorinated alkanes.

In a first aspect the invention relates to a composition comprising bacteria of *Desulfitobacterium* sp., said composition being capable of dehalogenating a haloalkane. This dehalogenation occurs under anaerobic conditions. The dehalogenation preferably occurs at a rate of at least 50 nmol Cl⁻ per mg bacterial protein per minute and/or occurs with a conversion efficiency of more than 50%. The haloalkane that is dehalogenated is preferably a halobutane, a halopropane or a haloethane. It is more preferably a dihaloalkane or trihaloalkane, even more preferably a haloalkane with vicinal halogen atoms. Preferred examples are selected from the group consisting of dihalobutane, dihalopropane, dihaloethane, trihalobutane, trihalopropane, and trihaloethane. Most preferred haloalkanes are chloroalkanes. Preferred dichloroalkanes and trichloroalkanes with vicinal halogens, dehalogenated by the composition of the present invention are 1,2-dichloroethane (1,2-DCA), 1,2-dichloropropane (1,2-D), 1,2-dichlorobutane (1,2-DCB), D-2,3-dichlorobutane (D-2,3-DCB), L-2,3-dichlorobutane (L-2,3-DCB), meso-2,3-dichlorobutane (meso-2,3-DCB), 1,1,2-trichloroethane (1,1,2-TCA), or mixtures thereof. The composition capable of dehalogenating haloalkanes can further comprise Vitamin K secreting living bacteria such as *Enterococcus sp.* or a medium conditioned by Vitamin K secreting living bacteria such as *Enterococcus sp.,* especially *E*. *casseliflavus* LMG 12901 and/or *E*. *faecalis* LMG 11207. Alternatively the composition can comprise Vitamin K from another source. The composition can further comprise a mixture of amino acids and vitamins such as vitamin B₁₂.

In a second aspect the invention relates to an isolated bacterium of the *Desulfitobacterium* genus characterised in having a 16S ribosomal DNA sequence as depicted in SEQ ID NO :1 or a 16S ribosomal DNA sequence being at least 97,0 % identical to SEQ ID NO:1. An example hereof is the *Desulfitobacterium dichloroeliminans* strain, deposited with accession number LMG P-21439.

In another aspect the invention relates to a medium for supporting the dehalogenation of haloalkanes by *Desulfitobacterium sp,* such as *D*. *dichloroeliminans,* characterised in that it comprises at least one Vitamin K, such as Vitamin K₁ or K₂. A preferred version of this medium for supporting dehalogenation of halalkanes is a medium that does not contain the *Desulfitobacteria* of the present invention. Such a medium can be obtained by conditioning with Vitamin K secreting living bacteria, such as with *Enterococcus sp.* under anaerobic conditions, or by adding Vitamin K to a standard bacterial growth medium.

In a third aspect the invention relates to a method for dehalogenating a haloalkane comprising the steps of, contacting an environment comprising said haloalkane with a composition comprising bacteria of *Desulfitobacterium* sp., preferably *D. dichloroeliminans,* capable of dehalogenating a haloalkane to in a quantity effective to dehalogenate said haloalkane and ensuring conditions supporting metabolism, growth and reproduction of the bacteria. In this method the haloalkane can be a halobutane, a halopropane or a haloethane. The haloalkane in this method can be a dihaloalkane or trihaloalkane. The haloalkane can be a haloalkane with vicinal halogen atoms. The haloalkane in this method can be a chloroalkane such as 1,2-dichloroethane (1,2-DCA), 1,2-dichloropropane (1,2-D), 1,2-dichlorobutane (1,2-DCB), D-2,3-dichlorobutane (D-2,3-DCB), L-2,3-dichlorobutane (L-2,3-DCB), meso-2,3-dichlorobutane (meso-2,3-DCB) and 1,1,2-trichloroethane (1,1,2-TCA). In this method the environment can be a contaminated site and the step of contacting the environment with the bacterial culture can occur in situ. In this method, ensuring conditions supporting metabolism, growth and reproduction can be done by maintaining or providing a pH value between 6 and 8 and maintaining or providing a temperature between 10 °C and 30 °C and maintaining or providing anaerobic conditions.

In a fourth aspect, the invention relates to a method for preparing a composition for supporting the dehalogenation by the Desulfitobacteria of the present invention comprising the steps of introducing a nutrient solution in an incubator vessel, making anaerobic conditions, sterilising the nutrient solution, optionally introducing an electron acceptor, introducing a bacterial culture of Vitamin K secreting bacteria such as *Enterococcus sp,*. and maintaining conditions suitable to allow growth and proliferation of the bacteria, and optionally cooling said vessel and contents to a temperature which prevents further growth of bacteria and which is suitable for storage of the culture. This method can be performed preferably with a culture of *Enterococcus* strains *E*. *casseliflavus* LMG 12901 and/or *E*. *faecalis* LMG 11207. Alternatively, the composition for supporting dehalogenation by the *Desulfitobacteria* of the present invention are made by adding a Vitamin K to a standard bacterial growth medium.

A fifth aspect of the invention relates to the use of a composition comprising bacteria of *Desulfitobacterium sp,* preferably *D. dichloroeliminans* and/or the use of a composition comprising a Vitamin K, in dehalogenating haloalkanes. This composition is preferably used for the remediation in situ on a contaminated site. This composition is more preferably performed under anaerobic conditions. Herein the haloalkane can be a halobutane, a halopropane or a haloethane such as 1,2-dichloroethane (1,2-DCA), 1,2-dichloropropane (1,2-D), 1,2-dichlorobutane (1,2-DCB), D-2,3-dichlorobutane (D-2,3-DCB), L-2,3-dichlorobutane (L-2,3-DCB), meso-2,3-dichlorobutane (meso-2,3-DCB) and 1,1,2-trichloroethane (1,1,2-TCA).

In a sixth aspect the invention relates to bacteria, being capable of dehalorespiring a haloalkane with vicinal halogen atoms under anaerobic conditions. In a preferred embodiment these bacteria are methanogenic or acetogenic bacteria. Preferably bacteria are chemotrophic or organotrophic. In another preferred embodiment these bacteria are anaerobic or aerotolerant anaerobic. A dehalorespiring bacterium that can dehalogenate a haloalkane can be identified by introducing a bacterium into a growth composition wherein the haloalkane is the sole electron acceptor.

### BRIEF DESCRIPTION OF THE FIGURES:

**Figure 1** represents a microscopic view (20.000 times magnified) of the novel bacterial strain *Desulfitobacterium dichloroeliminans* DCA1 (LMG P-21439) Scale bar represent 1 µm.
**Figure 2** represents the schematic conversion of perchloroethene to ethene via trichloroethene (TCE), dichloroethene (DCE) and vinylchloride (VC).
**Figure 3** represents a reaction scheme of the ATP coupled one-step dihaloelimination reaction performed by the *Desulfitobacteria* of the present invention, releasing completely dechlorinated end-products.

### DETAILED DESCRIPTION OF THE INVENTION

### Deposit of microorganism.

A culture of the bacterium *Desulfitobacterium dichloroeliminans* strain DCA1 has been deposited by Stefaan De Wildeman (Laboratory for Microbial Ecology and Technology (LabMET), Coupure Links 653, B-9000 Ghent Belgium) at the BCCM-LMG Bacterial Collection (University of Ghent, K.L. Ledeganckstraat 35, B 9000 Ghent, Belgium) on March 25, 2002 and was given the Accession number LMG P-21439). The viability was confirmed on March 25, 2002.

### Definitions.

"**Dehalogenation**" is the process whereby one or more halogen atoms (chlorine, bromine, fluor, iodine) are removed from a carbon chain. "**Dechlorination**" in the present invention refers to a particular form of "dehalogenation" and refers to the process whereby chlorine atoms are removed from chlorine carrying carbon atoms. In the present invention, the phrase 'capable of dehalogenating' preferably relates to bacteria which dehalogenate haloalkanes in the context of dehalorespiration but can also relate to bacteria which dehalogenate haloalkanes with a certain rate and efficiency as a result of genetic engineering or selection pressure.

"**Dehalorespiration**" in the present invention refers to the bacterial dehalogenation of halogenated hydrocarbons under anaerobic conditions, with hydrogen or formate as the direct electron donors and the halogenated substrates as the terminal electron acceptors. This is done in a respiratory process in anaerobic bacteria, which gain energy from this process. The only mechanism of dehalorespiration known is the reductive hydrogenolysis reaction. Reductive hydrogenolysis involves the substitution of a single halogen atom on a carbon atom by a hydrogen atom.

"**Dichloroelimination**" refers to simultaneous removal of two vicinal chlorine atoms on two vicinal carbon atoms, resulting in the production of a double bond.

"**Haloalkanes**" in the present invention refers to saturated organic compounds in which carbon atoms are substituted with one or more halogen atoms.

Haloalkanes of the present invention are haloalkanes with a indeterminate carbon chain length but preferably with a carbon chain length of less than 20, more preferably less than 15, even more preferably less than 10. Most preferably they are haloethanes, halopropanes and halobutanes.

The haloalkanes of the present invention are preferably di- and trihaloalkanes, most preferably di- and trichloroalkanes.

The haloalkanes of the present invention are more preferably molecules with vicinal halogens, most preferably vicinal chlorine atoms.

"**Haloalkenes**" refers to unsaturated organic compounds in which carbon atoms are substituted with one or more halogen atoms.

"**Vicinal**" refers to the presence of halogens on two adjacent carbon atoms of a haloalkane. Preferred examples of vicinal haloalkanes are 1,2-dichloroethane (1,2-DCA), 1,2-dichloropropane (1,2-D), 1,2-dichlorobutane (1,2-DCB), *D*-2,3-dichlorobutane (*D*-2,3-DCB), *L*-2,3-dichlorobutane (*L*-2,3-DCB), *meso-2,3*-dichlorobutane (*meso*-2,3-DCB), 1,1,2-trichloroethane (1,1,2-TCA). Other examples refer to the above-mentioned list wherein one or more chlorine atoms are replaced by other halogens, such as 1,2-dibromoethane (1,2-DBE) and 1,2 dibromopropane (1,2-DBP). Most preferred examples of vicinal haloalkanes are 1,2-dichloroethane (1,2-DCA) and 1,2-dichloropropane (1,2-D),

"**Chloroalkanes**" refers to saturated organic compounds in which carbon atoms are substituted with one or more chlorine atoms.

"**Chloroalkenes**" refers to unsaturated organic compounds from which carbon atoms are substituted with one or more chlorine atoms.

"**Anaerobic conditions**" in the present invention refers to conditions where no oxygen is present or to conditions in which oxygen is present but in which there is no excess of oxygen to inhibit metabolism of the bacteria of the present invention (i.e. substantially anaerobic conditions). Prolonged metabolism by the *Desulfitobacterium* of the present invention can be achieved at a redox potential below -180 mV.

"**Aerotolerant anaerobic bacteria**" refers to bacteria that in principle only metabolize in anaerobic conditions, but do tolerate high oxygen conditions during limited time periods. Tolerable high oxygen conditions for the bacteria of the present invention are for example the exposition to atmospheric conditions for at least 48 hours.

The standard concept of definition of "**Species**" for the purpose of taxonomy of bacteria is based on sequence comparisons of the gene for 16S ribosomal DNA. A bacterium is considered to represent a novel species in a genus when the 16S ribosomal DNA the bacterium species shows less that 97,0 percent in sequence identity with the most related 16S ribosomal DNA sequence of another species in the genus.

Accordingly, a "**Strain**" is assigned to a bacterium of a given species when the sequence identity of its 16S rDNA with the 16S rDNA from the type strain of that species is 97,0 % or more.

The genus ***"Desulfitobacterium"*** was originally described by Utkin et al. (1994) in Int. J. Syst. Bacteriol. 44:615 and consists of 4 species including *D. chlororespirans* (Sanford et al. 2001, Int. J. Syst. Bacteriol. 51:793), *D*. *dehalogenans* (Utkin et al. 1994, Int. J. Syst. Bacteriol. 44:615), *D*. *frappieri (* Bouchard et al. 1996, Int. J. Syst. Bacteriol. 46:1012) and *D*. *hafniense* (Niggemyer et al. 2001, Int. J. Syst. Bacteriol. 46:446). All four species are dehalorespiring bacteria, respiring PCE and/or TCE and/or an extended range of chlorophenols. However, not a single *Desulfitobacterium* was shown so far to be able to degrade haloalkanes. Based on the sequence analysis of the isolated *Desulfitobacterium* of the present invention, this culture represents a novel species (*D*. *dichloroeliminans*) of the *Desulfitobacterium* genus.

*Desulfitobacterium* bacteria of the present invention refer to bacterial cultures in solution or on growth plates but also to precipitates and pellets of bacteria obtainable from *Desulfitobacterium* comprising media or solutions. It further refers to dried, freeze dried, frozen (-180 or -70 °C) or cooled cultures of the bacteria of the present invention.

"**Composition**" in the present invention refers to both liquid as well as solid media. Examples of such liquid and solid media are bacterial growth media and buffered solutions, optionally rendered anaerobic by replacing oxygen with other gasses. Media and buffered solutions comprise for example electron donors, electron acceptors, carbon sources, amino acid mixtures, and one or more vitamins mentioned in the present specification in the example section.

"**Rate of dehalogenation**" is expressed as nanomoles of released halogen. This rate is determined under standard laboratory conditions (28°C). The bacterial protein concentration can be determined with a colorimetric assay such as the Coomassie assay described by Bradford (Bradford M. M. (1976) *Anal Biochem*. **72,** 248-254.). The released halogen content is calculated by measuring the concentration of halogenated substrates and dehalogenated product, which can be analyzed and quantified with headspace analysis or after extraction in hexane. In the case of 1,2-DCA, the concentration of the dechlorinated product ethene was measured by a gas chromatograph (Shimadzu Gas Chromatograph with a flame ionization detector and a packed column (2 m, Chrompack Porapaq N)) under isothermic conditions (80°C).

Rates of dehalogenation which are within the scope of the present invention are at least 100 nmol halogen ions per mg bacterial protein per minute, are preferably at least 200 nmol halogen ions per mg bacterial protein per minute, are even more preferably at least 300 nmol halogen ions per mg bacterial protein per minute, are most preferably at least 350 nmol halogen ions per mg bacterial protein per minute.

"**Conversion efficiency**" relates to the maximal percentage that can be degraded in a degradation process under conditions as described in the experimental section. Conversion efficiencies of the dehalogenation performed by the *Desulfitobacteria* of the present invention are more than 50%, preferably more than 70%, more than 80%, more preferably more than 90%, or even more preferably more than 95%.

"**Molar selectivity**" refers to the proportion of the end product versus possible intermediate products of the degradation product reaction. In the case of 1,2-DCA where only 2 % of 1,2 DCA is degraded to vinylchloride, the molar selectivity is 98%.

"**Effective quantities**" refer to amounts of bacteria of the present invention and may be dependent on the size of the sample, the amount of substrate and conditions such as pH and temperatures and can be determined experimentally. In a pilot experiment of reductive groundwater remediation (13 °C, pH 6,2) during 15 days, an effective quantity was the inoculation with 1 ppm of dry bacteria.

"**Bioremediation or biodegradation**" refers to the degradation of compounds by microorganisms. In the context of the present invention the remediation is performed using the dehalogenating bacteria of the present invention.

"**Site**" refers to aqueous media, such as groundwater, or contaminated soils comprising toxic or unacceptable levels of haloalkanes.

**"*in situ*"** in the context of bioremediation or biodegradation means that the compositions or bacteria of the present invention are introduced to or into the contaminated site. Thus there is no need for the removal of liquids and/or solid material which contain the compounds to be degraded.

"**Environment**" refers to, for example, but not exclusively to laboratory, industrial or in situ liquids and/or solid material which contain a compound to be degraded.

"**Vitamin K**" is a general term referring to a group of naphtoquinone derivatives required for the bioactivation of proteins involved in hemostasis. Vitamin K compounds are classified into three groups: Phylloquinones (Vitamin K₁) found in green plants; menaquinones (Vitamin K₂) primarily produced by intestinal bacteria and menadione (Vitamin K₃) which is a synthetic, lipid soluble precursor for other Vitamin K analogs.

In a first embodiment, the present invention relates to a novel bacterial culture of the *Desulfitobacterium* genus capable of dehalogenating a haloalkane, especially a haloalkane such as a chloroalkane under anaerobic conditions.

In a preferred embodiment, the present invention relates to the novel bacterial culture *Desulfitobacterium* genus capable of dechlorinating a dichloroalkane or a trichloroalkane. Preferably, said dichloroalkane or trichloroalkane has vicinal chlorine atoms.

Even more preferred the present invention relates to a bacterial culture of the *Desulfitobacterium* genus capable of dechlorinating dichloroalkanes or trichloroalkanes with vicinal chlorine atoms selected from the group comprising 1,2-dichloroethane (1,2-DCA), 1,2-dichloropropane (1,2-D), 1,2-dichlorobutane (1,2-DCB), *D*-2,3-dichlorobutane (*D*-2,3-DCB), *L*-2,3-dichlorobutane (*L*-2,3-DCB), *meso*-2,3-dichlorobutane (*meso*-2,3-DCB), 1,1,2-trichloroethane (1,1,2-TCA) or mixtures thereof.

In a further embodiment, the present invention relates to a bacterial culture of *Desulfitobacterium dichloroeliminans* that has the characteristics of the allocated accession number LMG P-21439.

According to the present invention the novel bacterial species of the *Desulfitobacterium* genus is capable of fully dehalogenating vicinal haloalkanes and especially dechlorinating vicinal dichloroalkanes and dechlorinating 1,1,2-trichloroethane to vinylchloride. The major advantages of this novel bacterial *Desulfitobacterium* species include the capacity of complete dehalogenation and especially dechlorination of toxic compounds such as dichlorinated alkanes in a very rapid way (>250 nmol Cl⁻ /mgₚᵣₒₜₑᵢₙ/min) and with a surprisingly high conversion efficiency (>99.5% conversion).

Sequence comparison of the 16S rDNA fragment depicted in SEQ ID NO:1 of strain DCA1 revealed a 96.6% sequence identity with the corresponding gene of its closest relative in the taxonomic tree, *Desulfitobacterium hafniense.* As the closest relation was below the limit of 97,0 % identity, and as *D. dichloroeliminans* cannot dehalorespire PCE nor chlorophenols, in contrast to other *Desulfitobacteria,* the present invention describes a novel bacterial species. Thus, the isolated *Desulfitobacterium dichloroeliminans* of the present invention belongs to this novel species of *Desulfitobacterium.*

*Desulfitobacterium dichloroeliminans* has several typical physiological and morphological characteristics (see also example 2). Morphologically, the DCA1 bacteria are gram positive, motile, non-sporulating curved rods of 0.5-0.7 to 2-5 µm (Figure 1). Occasionally, cell lengths may exceed 10 µm. Optimal pH and temperature for growth are within the range of respectively 7.2 - 7.8 and 25 - 30°C.

*Dehalococcoides ethenogenes* strain 195 and the *Desulfitobacterium dichloroeliminans* of the present invention are capable of dechlorination of 1,2 DCA under anaerobic conditions. However, *D. dichloroeliminans* lacks several of the disadvantageous characteristics of *Dehalococcoides ethenogenes* strain 195.

*Desulfitobacterium dichloroeliminans* shows an expanded versatility of electron donors and acceptors. Optimal dehalogenation, especially dechlorination performance can be obtained with the electron donors H₂, formate, lactate and pyruvate. Electron acceptors usable by *D. dichloroeliminans* include several vicinal dihaloalkanes, NO³⁻, SO₃²⁻ and S₂O₃²⁻. For comparison, growth of *Dehalococcoides ethenogenes* strain 195 can only be sustained by the electron donor H₂, and besides the chlorinated compounds that fit the dehalogenases of the *Dehalococcoides ethenogenes* strain 195, no other electron acceptors can be used for respiration. In addition, *D. dichloroeliminans* is tolerant to up to 12 g/l of NaCI, which may facilitate tracer tests.

Surprisingly, *D. dichloroeliminans* is very oxygen tolerant and can thus be considered as aerotolerant anaerobic. For instance, it can start 1,2-DCA dechlorination after inoculation in a mixed bacterial culture exposed to aerobic conditions. *D. dichloroeliminans* can survive a 1-day exposure to air. For comparison, *Dehalococcoides ethenogenes* strain 195 irreversibly loses its dechlorination activity after exposure to aerobic conditions for some seconds. Finally, *D*. *dichloroeliminans* has a significantly higher selectivity for ethene production and a higher conversion rate compared to *Dehalococcoides ethenogenes* strain 195.

Several of these characteristics are important tools to allow easy and inexpensive preparation of a *Desulfitobacterium* DCA1 culture and its application in bioremediation. Table 1 compares several characteristics of *D*. *dichloroeliminans* with *Dehalococcoides ethenogenes* strain 195.

**Table 1:**

| ***Characteristics of Desulfitobacterium dichloroeliminans strain DCA1 and Dehalococcoides ethenogenes strain 195*** | | |
|---|---|---|
| | *Desulfitobacterium dichloroeliminans* strain DCA1 | *Dehalococcoides ethenogenes strain* 195 |
| microscopic study | curved rods (2-5 µM) | small cocci (0.5µM) |
| 1,2 DCA conversion | 5-56 µmol / (L.h) | 2-36µmol / (L.h) |
| rate | ∼100% | ∼100% |
| conversion | >99.8 % | >99.0% |
| selectivity ethene selectivity VC | 0.03% | 1% |
| Oxygen tolerance | survives at least 24h in air | survives a few seconds in air |
| Electron donors | H₂-acetate | H₂-acetate |
| | lactate, formate-acetate, pyruvate | |
| Electron acceptors | NO³⁻, SO₃²⁻, S₂O₃²⁻ 1,2-DCA, 1,2-D, 1,2-DCB, *D*-2,3-DCB, *L*-2,3-DCB, *meso*-2,3-DCB. 1,1,2-TCA | No inorganic electron acceptors PCE, TCE, *cis* 1,2-DCE, VC, 1,2-DCA |

As mentioned above, the *Desulfitobacterium* of the present invention is capable of dehalogenating 1,2 DCA under anaerobic conditions. In addition, several other dichloroalkanes can be fully dehalogenated. For example, *Desulfitobacterium* strain DCA1 can fully dechlorinate 1,2-D, 1,2-DCB, *D*-2,3-DCB, *L*-2,3-DCB and *meso*-2,3-DCB. 1,1,2-TCA is dechlorinated to vinylchloride.

In another embodiment, the present invention relates to a bacterial culture of the *Desulfitobacterium* genus, more preferably strain DCA1, which is capable of performing a dichloroelimination.

In another embodiment the invention also relates to *Desulfitobacterium* species, which can dehalogenate haloalkanes with vicinal tri and dihalogens. Such a species can be a natural isolate and can be detected by the methodology described in example 1 using a substrate such as 1,2-DCA as sole electron acceptor. In this context it also refers to *Desulfitobacterium* species and strains which have been subjected to chemical or physical (e.g. radiation) mutagenesis in order to obtain a modified dehalogenase with haloalkane dehalogenation activity.

In another embodiment the invention also relates to compositions comprising mixtures of different bacterial species wherein the *Desulfitobacteria* of the present invention are the predominant bacteria performing dehalogenation of haloalkanes. Preferably the invention relates to compositions that are obtained from an isolated culture of the bacteria of the present invention.

In tests with several di- or trichlorinated substrates with vicinal chlorine atoms, *D*. *dichloroeliminans* was shown to convert 1,2-DCA, 1,2-D, 1,1,2-TCA, 1,2-DCB, *D*-2,3-DCB, *L*-2,3-DCB, *meso*-2,3-DCB using a similar and highly selective dihaloelimination mechanism. In fact, *D. dichloroeliminans* is the first dehalorespiring bacterium isolated so far that exclusively dihaloeliminates its substrates. All possible vicinal dichloroethanes, -propanes and -butanes are converted to the corresponding alkenes in one rapid dihaloelimination step.

Although "reductive hydrogenolysis", i.e. the substitution of a single halogen atom on a carbon atom by a hydrogen atom under anaerobic conditions, is the most important mechanism of dehalorespiration known today, the *Desulfitobacterium* bacteria of the present invention follow another, more efficient dehalogenation process. Theoretical calculations on the Gibbs free energy that is involved in reductive hydrogenolysis and dihaloelimination show the advantage of the latter reaction mechanism. For example, dichloroelimination of 1,2-DCA yielding ethene delivers more energy than a reductive hydrogenolysis yielding chloroethane.

In another embodiment, the present invention relates to a bacterial culture of the *Desulfitobacterium* genus, more preferably *D. dichloroeliminans,* which performs the dehalogenation process with a conversion efficiency of more than 50%, preferably more than 70%, more than 80%, more preferably more than 90%, or even more preferably more than 95%.

For example, *Desulfitobacterium dichloroeliminans* of the present invention can almost totally, i.e. for 99.5%, convert 1,2 DCA with a surprisingly high molar selectivity of at least 99.8%. This means that upon conversion less than 0.2% VC will remain. For comparison, *Dehalococcoides ethenogenes* strain 195 is able to reach molar selectivity of 99%, and thus upon conversion of 1,2-DCA still produces 1% VC. Thus, *Desulfitobacterium dichloroeliminans* combines full dehalogenation capacity with an elevated molar selectivity and a high rate one step reaction, which renders the dehalogenation process performed by this species very efficient.

In another embodiment, the present invention relates to a bacterial consortium comprising
(a) a bacterial culture of *Desulfitobacterium sp.* according to the invention, preferably *D*. *dichloroeliminans,* capable of dehalogenating a haloalkane, preferably a dichloroalkane or a trichloroalkane, and even more preferably a dichloroalkane or a trichloroalkane that has vicinal chlorine atoms, under anaerobic conditions; and
(b) a bacterial culture of a Vitamin K secreting bacteria such as *Enterococcus sp.*

A "bacterial consortium" can be defined as a mixture of different bacteria. In a more preferred embodiment, the bacterial consortium of the invention comprises a bacterial culture of *Desulfitobacterium dichloroeliminans* strain DCA1 (LMG P-21439) and a bacterial culture of *Enterococcus casseliflavus* (LMG 12901) or *Enterococcus faecalis* (LMG 11207) or mixtures thereof.

In particular the bacterial consortium of the invention is capable of dechlorinating di-or trichloroalkanes that have vicinal chlorine atoms and that are selected from the group consisting of 1,2-DCA, 1,2-D, 1,2-DCB, *D*-2,3-DCB, *L*-2,3-DCB, *meso*-2,3-DCB and 1,1,2-TCA, or mixtures thereof.

In another embodiment, the present invention relates to a method for preparing a bacterial consortium comprising the steps of:
- imposing a nutrient solution in an incubator vessel,
- making anaerobic conditions,
- sterilizing said nutrient solution,
- introducing an electron acceptor,
- introducing a bacterial culture of the *Desulfitobacterium* genus according to the invention,
- introducing a bacterial culture of a Vitamin K secreting bacteria such as *Enterococcus sp.*
- maintaining conditions suitable to allow growth and proliferation of the bacteria; and
- optionally, cooling said vessel and contents to a temperature which prevents further growth of bacteria and is suitable for storage of the culture.

More preferably, the present invention relates to a method for preparing a bacterial consortium whereby the bacterial culture of the *Desulfitobacterium* genus is a culture of *D. dichloroeliminans* strain DCA1 (LMG P-21439) and the bacterial culture of the *Enterococcus* genus is a culture of *Enterococcus casseliflavus* strain LMG 12901 and/or *Enterococcus faecalis* strain LMG 11207.

The nutrient solution introduced in the incubator vessel may comprise a pH indicator such as resaruzin, a basal medium comprising different salts and water. Also a yeast extract and pyruvate as preferred electron donor are added. Anaerobic conditions are installed by using N₂ or Ar gas, according to method known by a person skilled in the art, and the medium is sterilised. A suitable electron acceptor may comprise 1,2-DCA, 1,2-D, 1,2-DCB, 1,3-DCB *(meso* and *dl* forms), 1,1,2-TCA, or S₂O₃²⁻. Introduction of a bacterial culture of the *Desulfitobacterium* genus according to the invention and of a bacterial culture of a Vitamin K secreting bacterium such as *Enterococcus* may comprise the inoculation of a 2-5 vol %, more preferred a 1 vol%, of the respective cultures in the incubator vessel.

Preferably, in order to test the viability of the bacterial consortium and its dechlorination capacity, 1,2-DCA and S₂O₃²⁻ are added to the prepared consortium and degradation of these electron acceptors is monitored. In order to obtain a thicker growth of the bacteria in the culture, pyruvate may be added as a fermentation substrate.

The major conditions and products, which are needed for the preparation and growth of the bacterial consortium of the present invention include a low-cost C/N/P source, and the presence of an electron donor, which can be selected from H₂-acetate, lactate, formate-acetate or pyruvate. A pH of 7-8 is preferred, pH values between 6 and 7 slow down the dehalogenation with a factor 2 to 3 but are still acceptable for industrial applications. In view of these operational conditions it is clear that the costs required for preparing the subject bacterial consortium are minimal. Using other electron donors such as the addition of SO₃²⁻, S₂O₃²⁻ or NO₃⁻ requires the addition of a vitamin mixture.

The method for production of a bacterial consortium provides a bacterial consortium comprising *Desulfitobacterium sp.* at a density preferably comprised between 10⁸ and 10¹⁰ bacteria /ml, with *Enterococcus* cells not exceeding 10⁸ bacteria /ml. Injection of 1 vol% of this culture results in the presence of at least 10⁻⁶ DCA1 bacteria /ml in the contaminated water. Corresponding to these amounts, the high dechlorination rate is guaranteed when at least 1 mg/l bacterial protein of *d. dichloroeliminans* is present.

The optimal ratio *Desulfitobacterium* over *Enterococcus* cells in the consortium according to the invention may be comprised between 10 and 1000.

The bacterial consortium prepared according this method is effective at temperatures ranging from 10 to 20 °C. For instance, injection of a 1 vol% inoculum of this thick grown culture in groundwater containing 400 µM of 1,2-DCA under laboratory conditions results in a full detoxification within 1 week at 18°C. This inoculum is also able to dechlorinate 400 µM 1,2-DCA containing groundwater in the presence of a technical grade lactate within 10 days at 13°C.

In another embodiment, the bacterial consortium may be prepared according to the invention and further processed. For example, the consortium may be centrifuged in order to obtain a pellet consisting of humid cell mass, which may be further dried. According to this process, humid or dried pellets of the bacterial consortium of the invention can be obtained. Such pellets contain the nutrients suitable for the bacteria of the consortium to remain viable and active. Preparation of pellets of the bacterial consortium makes it possible to obtain the bacterial consortium of the invention in a user-friendly and easy-to-handle way. These humid or dried pellets may be advantageously used in various applications, for instance for the *in situ* remediation of contaminated sites or for industrial applications.

The above-produced bacterial consortium can thus effectively be used to remediate samples from sites contaminated with haloalkanes. Therefore, in another embodiment the present invention relates to the use of the bacterial consortium according to the invention for *in situ* remediation of a site, preferably under anaerobic conditions. Preferably, the site is contaminated with at least one haloalkane, more preferably one or more di- or trichloroalkanes, especially those that have vicinal chlorine atoms.

The present invention therefore also relates to a method for remedying a site contaminated with chloroalkanes under anaerobic conditions comprising the steps of:
- introducing into said site a bacterial consortium according to the invention in a quantity effective to remediate said halooalkane in said site; and
- if necessary, maintaining conditions for growth and dehalogenation by said bacterial consortium.

In a preferred embodiment, the invention relates to a method for degrading a chloroalkane with vicinal chlorine atoms. More preferably, a di- or trichloroalkane with vicinal chlorine atoms is selected from the group comprising 1,2-DCA, 1,2-D, 1,2-DCB, *D*-2,3-DCB, *L*-2,3-DCB, *meso*-2,3-DCB, 1,1,2-TCA or mixtures thereof.

The amount of the bacteria in the bacterial consortium must be effective to result in dehalogenation of the contaminants to the desired level. The amounts will therefore vary and may be readily determined by one of ordinary skill in the art, depending on the efficacy of the bacterial consortium and the level of decontamination required.

In another embodiment, the said bacterial consortium is prepared according to the above-described method. Once a suitable bacterial consortium has been produced, the soil, sediments, and/or water of a contaminated site is remediated by inoculation of the site with the bacterial consortium by methods known by those of ordinary skill in the art. For example injection wells or other forms of conduits can be used. Since the bacterial consortium functions in anaerobic conditions, injection preferably occurs in an anaerobic zone of the contaminated site. Anaerobic conditions may be detected, for example, by measurement of a low dissolved oxygen and a negative oxidation-reduction potential in water from the zone, as disclosed by E. J. Bouwer in "*Handbook of Remediation*", Norris R. D. et al., (Eds.), Lewis Publishers, 1994, pp. 149-175, which is incorporated by reference herein.

In a preferred embodiment, the invention relates to a method whereby conditions supporting growth and reproduction of said bacterial consortium in said site are provided or maintained. The quantity of microbes and the efficacy of the consortium may be affected by adjusting or maintaining at least one site parameter. Exemplary parameters include, but are not limited to, pH, electron donor or nutrient level, oxygen level, rate of flow of the aquifer, and level of toxic or inhibitory compounds. Adjustment is by means known in the art, for example, electron donors such as organic acids, sugars, alcohols, or other suitable carbon-containing substrates and other nutrients may be injected or otherwise added to the subsurface to stimulate bacterial activity and cause the aquifer to become anaerobic. Anaerobic conditions may be increased or maintained by pumping nitrogen or other anoxic gases to the zone of remediation. The temperature in the contaminated site may be comprised between 10°C and 20°C. Temperatures within this range will not compromise the dehalogenation capacity of the introduced bacterial consortium.

Bioremediation according to the invention is particularly advantageous for the treatment of contaminated sites where suitable indigenous microbial populations are either not present, or are present at concentrations ineffective for the remediation of haloalkanes. There are a number of reasons why a site may be unable to support the appropriate microbial growth; for example, the site may have been exposed to the contaminant for an insufficient time to allow adaptation and growth, or may be insufficiently anaerobic. This method is also particularly advantageous where the speed of decontamination is a consideration. Adding a bacterial consortium with known biodegradative capabilities can be used to start the remediation process with little or no lag time. It is also advantageous where little is known about the site other than the original source of contamination, and little money is available for testing. Inoculation with an appropriate bacterial consortium assures that the proper microbes are present in sufficient numbers to destroy the contaminant.

In another embodiment of the present invention, the living vitamin K secreting bacteria of the consortium can be replaced by a conditioned medium of vitamin K secreting bacterium or can be replaced by a vitamin K supplement.

In another embodiment the present invention relates to a new dehalogenase enzyme catalyzing a selective *anti* dichloroelimination. In a preferred embodiment, the dehalogenase enzyme catalyzing a selective *anti* dichloroelimination process isolated from bacteria of the *Desulfitobacterium* genus, more preferably from *Desulfitobacterium* strain DCA1 (LMG P-21439). More particularly, the dichloroelimination reaction catalyzed by the dehalogenase enzyme of the present invention is stereochemically interesting because of its high selectivity for anti elimination. The stereochemical selectivity of the dehalogenase of the present invention was elucidated by the examination of dechlorination products of the stereoisomers of 2,3-dichlorobutane (*meso*, *D* and *L* forms), as described in example 2.

The only dehalogenase known to perform a dichloroelimination reaction in addition to reductive hydrogenolysis, is the TCE dehalogenase of *Dehalococcus ethenogenes* strain 195, having 1,2-DCA as the only dichloroelimination substrate. However, this TCE dehalogenase is limited especially in terms of selectivity. Strain DCA1 developed a novel biocatalytic dehalogenase, effectively dichloroeliminating its fitting chlorinated substrates in a one step process. The dehalogenase of strain DCA1 is highly selective, as it does not convert any chlorinated ethenes. Moreover, the molar dichloroelimination selectivity for all of the extended range of other chloroalkanes, exceeds 99.8%. Furthermore, the inhibition effect of chloroform, a known inhibitor of chlorinated ethene dehalogenases, is much lower compared with other dehalogenase enzymes.

Due to its very unique and specific activity, the dehalogenase enzyme of strain DCA1 can be very useful in several industrial processes related to dechlorination of chloroalkanes in general. The dehalogenase enzyme of strain DCA1 can be used as a catalyst. It may be especially suitable for dechlorination reaction of dichloroalkanes or trichloroalkanes, in particular with vicinal chlorine atoms. Use of the dehalogenase enzyme of the present invention can result in more rapid and energetically more efficient dichloroelimination processes. Therefore, in a further embodiment, the present invention relates to the use of the dehalogenase enzyme according to the invention, a bacterial culture according to the invention or a bacterial consortium according to the invention, as a catalyst for enzymatic detoxification. More particularly, enzymatic detoxification comprises dehalogenation, or even more preferred dechlorination. The dechlorination may comprise the dechlorination of a chloroalkane, preferably a di-or trichloroalkane, more preferably a di-or trichloroalkane having vicinal chlorine atoms.

The dehalogenase enzyme can be isolated from bacterial lysates with classical isolation procedures (such as ammoniumsulphate precipitation, ion exchange chromatography and gel filtration). Fractions can be isolated for their ability to dehalogenate e.g. 1,2-DCA, which is preferentially preferred under strong reducing conditions (for example in the presence of titanium citrate).

Isolation of the DNA sequence of the dihaloeliminating dehalogenase can be performed by designing degenerated primers from dehalogenase enzyme DNA sequences with closely related substrate specificity and/or from bacteria of related phylogenetic background. These primers can be used to screen libraries or can be used to perform PCR reactions on bacterial DNA. A successful strategy to isolate putative bacterial pentacholorophenol dehalogenase DNA sequences is described in Villemur et al . (2002) *Can J. Microbiol*. **48,** 697-706. The cloned gene can then be expressed and isolated by recombinant technology. Alternatively the gene can be cloned and transfected into a microorganism, whereafter this transfected organism is used to perform in vivo dehalogenation.ln another embodiment, the invention also relates to a method for the enzymatic degradation of chloroalkanes by using the dehalogenase enzyme of the invention. The method of the invention provides a novel, unique, and clean approach for degradation of large amounts of chloroalkanes, by using an enzyme which catalyzes the simultaneous removal of two vicinal chlorine atoms on two vicinal carbon atoms. In addition, the present invention relates to a method showing a high selectivity for *anti* elimination. No *anti* elimination reactions on vicinal dichloroalkanes have been reported in mild aqueous conditions at ambient temperatures.

In an embodiment, the method for enzymatic dechlorination of at least one chloroalkane under anaerobic comprises the steps of:
- contacting said chloroalkane to a dehalogenase enzyme according the invention; or a bacterial culture according to the invention, or a bacterial consortium according to the invention, and thereby
- dechlorinating said chloroalkane employing said enzyme.

Preferably, said chloroalkane is a dichloroalkane or a trichloroalkane. More preferably, said dichloroalkane or trichloroalkane has vicinal chlorine atoms and may be selected from the group comprising 1,2-DCA, 1,2-D, 1,2-DCB, *D*-2,3-DCB, *L*-2,3-DCB, *meso*-2,3-DCB, 1,1,2-TCA, or mixtures thereof.

Practically, the enzyme may be processed to a suitable catalyst form, for instance a gel or a matrix, according to techniques known in the art. Alternatively the dehalogenase enzyme according to the invention may also be used in the form of a bacterium producing the enzyme. This bacterium may be selected from the *Desulfitobacterium* genus. More preferably, the bacterium is *Desulfitobacterium dichloroeliminans* strain DCA1. In another alternative, also a bacterial consortium according to the invention and comprising bacteria producing the dehalogenase enzyme according to the invention may be used. The catalyst can for example be introduced in a bioreactor. Subsequently, the bioreactor is fed with particular rates of chloroalkanes. Suitable temperature and pressure conditions are applied in order to allow the catalyzed conversion of the chloroalkanes.

In conclusion, the invention discloses a novel type of dehalorespiring bacteria exemplified as strain DCA1. This strain belongs to a novel *Desulfitobacterium* species, called *Desulfitobacterium dichloroeliminans*. This particular strain expresses a dehalogenase enzyme which catalyzes a highly selective anti dihaloelimination in aqueous solution. Strain DCA1 exclusively relies on the dihaloelimination of vicinal dihaloalkanes. Due to its unique dehalogenation capabilities, this bacterium can be effectively used for full dehalogenation and detoxification of important vicinal halogenated, especially chlorinated groundwater pollutants. In addition, given its strong biocatalytic dehalorespiration mechanism, this bacterium or its isolated enzyme can also be used as an efficient reductive biocatalyst for the conversion of haloalkanes such as dichloroalkane and trichloroalkanes with vicinal chlorine atoms in several industrial applications. As mentioned the preferred application is the in situ remediation of contaminated sites under anaerobic conditions. The invention can however also be applied directly to waste solvents of industrial processes comprising di- and trihalogenated alkanes, when these solvents are rendered anaerobic, supplemented with nutrients and electron donors and electron acceptors and inoculated with the bacteria of the present invention as disclosed in the present document.

While preferred embodiments have been shown and described, various modifications and substitutions may be made thereto without departing from the spirit and scope of the invention. Accordingly, it is to be understood that the present invention has been described by way of illustration and not limitation. Following non-limiting examples support several aspects of the invention.

### EXAMPLE 1 Isolation of Desulfitobacterium dichloroeliminans strain DCA1 from a site contaminated with 1,2-DCA

The bacterial culture of the present invention was obtained by laboratory culturing of the indigenous microbes present at a site contaminated with at least one chlorinated hydrocarbon, by using methods known by those of ordinary skill in the art. The starting bacterial inoculum was obtained from the soil matrix of an anoxic water-saturated layer (1 m depth) that had been exclusively polluted with 1,2-DCA (50 mg/kg) for 30 years (soil under industrial storage tank containing 1,2-DCA). No other chlorinated substrates were present. The inoculum (1% w/v) was transferred to sterilized anaerobic media containing 40 mM electron donor, 400 µM 1,2-DCA (sole electron acceptor; for reasons of toxicity, concentrations of more than 1 mM 1,2-DCA are avoided during the initial exponential phase), 0.02% (w/v) Yeast Extract (YE), vitamins (300 nM vitamin B₁, 800 nM vitamin B₃, 40 nM vitamin B₇, 40 nM vitamin B₁₂, 300 nM vitamin H₁, 100 nM Ca-D(+)-panthotenate, 600 nM pyridoxamin.2HCl), trace elements (20 nM N₂SeO₃.5H₂O, 10 nM Na₂WO₄.2H₂O, 7.2 µM FeSO₄.7H₂O, 1 µM ZnCl₂, 0.2 µM boric acid, 20 nM CuCl₂.2H₂O, 0.2 µM NiCl₂.6H₂O, 0.3 µM Na₂MoO₄.2H₂O), salts (0.2 mM MnCl₂.4H₂O, 0.5 mM Na₂SO₄, 1 mM CaCl₂.2H₂O, 7 mM KCI, 16 mM NaCI, 2 mM MgCl₂.6H₂O, 5 mM NH₄Cl, 2 mM KH₂PO₄), cysteine.HCl (1 mM), resazurin (2 mg/l) and NaHCO₃ (50 mM). After enrichment and isolation, suitable electron donors (40 mM) for strain DCA1 were H₂ (40 mM nominal concentration), formate and lactate. Media with H₂ or formate additionally contained 5 mM acetate (carbon source). Substituting YE by a defined mixture of amino acids (e.g. R7131 RPMI-1640, Sigma-Aldrich, Belgium) and supplying 1 µM Vitamin K₁ or Vitamin K₂ (stock solution of 5 mM in pure isopropanol) allowed indefinite subcultivation of strain DCA1 under completely defined conditions.. Initial electron donors were pyruvate, lactate, formate, butyrate, H₂ and ethanol. Of these electron donors, only pyruvate sustained growth, as it can function both as electron donor and electron acceptor. However, even with pyruvate, as with all other substrates, no dechlorination could be observed, as pyruvate is used preferentially over 1,2-DCA as electron acceptor. A transfer of pyruvate grown bacteria into fresh lactate, formate, butyrate, H₂ and ethanol containing media resulted in an almost complete removal of the 400 µM 1,2-DCA within 6 to 21 days since omitting pyruvate forces the bacterium to use 1,2-DCA as electron acceptor.

The conversion products of 1,2-DCA were found in stoechiometric amounts as ethene and HCI. After a second 10 vol% transfer, 0.02% of yeast extract was needed for sustainable dechlorination. Six more weekly 5 vol% transfers were done, subsequently enhancing the dechlorination rate of 400 µM 1,2-DCA. After these 9 transfers, the 1,2-DCA dechlorination rate stabilized in the absence of methanogenesis and acetogenesis. Accelerating 1,2-DCA conversion rates exceeding 1.3 mM/day (130 mg 1,2-DCA/liter medium/day) were observed when the culture was subsequently respiked with accumulating 1,2-DCA concentrations, indicating metabolic conversion. A denaturing gradient gel electrophoresis (DGGE) pattern of this enrichment culture indicated the presence of 3 dominant eubacterial species in the sediment-free enrichment culture. A PCR amplification with specific *Dehalococcoides* primers on DNA extracts from the enrichment culture showed that the bacterial enrichment culture did not contain *Dehalococcoides* bacteria.

Isolation was started from this enrichment culture. Selective agar compositions (3% w/w) containing 1,2-DCA were prepared in roll-tubes. Dechlorination activity in the tubes was confirmed by the production of ethene. Transfer of colonies to liquid media resulted in a dechlorination activity in about 3% of all transfers. These dechlorinating cultures contained 2 different bacteria: a coccoid bacterium and a curved rod. In the other 97% of the transferred colonies, a non-dechlorinating coc or straight rod was isolated. After physiological characterization, the curved rod in the bibacterial culture was designated as the dechlorinating strain. The curved rod could outcompete the coc by at least 2 orders of magnitude in the presence of pyruvate and 1,2-DCA. Liquid medium dilution series of this bibacterial culture indicated a symbiotic interaction between the coc and the curved rod. Indeed, only growth and dechlorination sustaining media contained both bacteria.

To further separate colonies of the curved rod, dilution series of the enriched bibacterial culture were made in liquid media. The bibacterial culture was grown in separate media till dechlorination activity was started. Hence, all nutrients sustaining dechlorination and growth of the curved rod were present. Afterwards, these media were pasteurized and used in new dilution series. In the conditions tested, none of both bacteria built endospores. No other contaminating bacteria could be detected by cultivation in roll-tubes containing 0.1% yeast extract, 40 mM pyruvate or 40 mM glucose, or by aerobic cultivation on a medium which supports the growth of a wide variety of microorganisms including those having complex nutritional requirements (for example brain heart infusion agar). The curved rod was successfully isolated in these dilution series and was designated *Desulfitobacterium strain* DCA1 (Figure 1, see also example 2). The non-dechlorinating symbiotic coccoid bacterium was characterized as an *Enterococcus casseliflavus* strain.

In conclusion, a bacterial consortium comprising *Desulfitobacterium strain DCA1* and an *Enterococcus casseliflavus* strain could be isolated from a site contaminated with 1,2-DCA.

### EXAMPLE 2 Phylogenetic, morphological and physiological characterization of Desulfitobacterium strain DCA1

Transfer of 50 vol% sterile filtered supernatant of the pure symbiotic *Enterococcus casseliflavus* strain, which was cultivated in the presence of 0.1% yeast extract, allowed the preparation of cell-free media in which physiological characterization of strain DCA1 can be studied. Present example provides an overview of the typical characteristics of the novel *Desulfitobacterium* strain DCA1. The bacterial strain was deposited with Accession number LMG P-21439.

Phase-contrast and scattering electron microscopy of the motile Gram positive strain DCA1 revealed curved rods of 0.5-0.7 µm diameter to 2-5 µm length (Figure 1). Occasionally, cell lengths exceeding 10 µm can be observed. Procedures to induce endospore formation were applied, but no viability could be shown after pasteurisation. Small transparent colorless colonies (<2 mm diameter) could be obtained in roll-tubes with 1,2-DCA, 3% agar, 0.02% yeast extract, a supplement solution and lactate (40 mM) or H₂/CO₂ (90/10)-acetate (5mM) when *E*. *casseliflavus* was present as a co-culture. Omitting 1,2-DCA did not result in colony growth of strain DCA1.

Comparisons of the nucleotide base sequence of the gene of 16S ribosomal DNA between different species indicate the degree to which they are related to each other. Comparison of a stretch of 1467 nucleotides of the 16S rDNA of strain DCA1 (SEQ ID NO:1) revealed a 96.6% sequence identity with the corresponding gene in the closest relative, *Desulfitobacterium hafniense* DSM 10664^{T}. Due to reproducible double signals in the first 150 basepairs, this region of the sequence was not included in this calculation. Hence a theoretical similarity of at most 96.9% can be obtained when this region would be identical to that of *D*. *hafniense* DSM 10664.

*Desulfitobacterium* strain DCA1 can grow on several electron donors and electron acceptors. With the electron donors lactate and H₂, no growth occurred in the absence of electron acceptors. Strain DCA1 grew in the presence of 3mM SO₃²⁻, S₂O₃²⁻ or NO₃⁻. Fumarate, SO₄²⁻, NO₂⁻ and O₂ were not converted as electron acceptors. Fumarate and SO₄²⁻ were not toxic, while NO₂⁻ fully inhibited the dechlorination at 2.5 mM.

When electron donors were omitted but 1,2-DCA and 0.02% yeast extract media were present, growth started but rapidly slowed down. Additional electron donors as H₂ (50 mM nominal concentration), lactate (40 mM) or pyruvate (40 mM), sustained extended growth dichloroeliminating up to 6 mM of 1,2-DCA. Formate and glucose did not sustain growth of strain DCA1. The supernatant was also needed for growth on all 3 inorganic electron acceptors, indicating an excretion product of the isolated *E*. *casseliflavus* strain. Aerobically grown *Enterococcus* cells did not excrete the essential compound. It was found that this component secreted under anaerobic conditions was Vitamin K. The supernatant of both E. *casseliflavus* and *E*. *faecalis* strains allowed growth of strain DCA1, while that of the *E*. *avium* strain did not. It has been reported that both *E*. *casseliflavus* and *E*. *faecalis* species produce menaquinones, in contrast to *E*. *avium* (Collins M.D. and Jones D. (1979). *Journal of General Microbiology* **114,** 27-33.). As an alternative for *Enterococcus,* other Vitamin K secreting bacteria can be used. Well-known examples of Vitamin K producing bacteria are lactid acid bacteria (Morishita et al. (1999) *J. Dairy Sci.* **82**,1897-1903) and a large number of anaerobic intestinal bacteria (Ramotar et al. (1984) *J*. *Inf*. *Dis.* **150,** 231-218). Eventually these bacteria can be cocultured with strains belonging to the novel species *Desulfitobacterium dichloroeliminans.* The supernatant can be replaced by yeast extract or another growth medium for microbiology such as peptone or tryptone, or a mixture of amino acids such as R7131 RPMI-1640, Sigma, Belgium), which medium or amino acid mixture is supplemented with vitamin K and other vitamins (p-amino-benzoic acid (Vitamin H1), D(+)-biotin (Vitamin B7), Nicotinic acid (Vitamin B₃), Vitamin B_{12,} Ca D(+)-panthotenic acid, Pyridoxamin, Thiamin. HCI (Vitamin B₁).

Surprisingly, strain DCA1 is not highly oxygen sensitive. It can start 1,2-DCA dechlorination after inoculation in a mixed bacterial culture exposed to aerobic conditions. After a lag phase of at least 24 hours, the redox potential of this medium decreased below -180 mV, which appears needed for 1,2-DCA dechlorination. Vitamin B₁₂ is needed for growth and for sustainable dechlorination activity of strain DCA1. In a first 5 vol% transfer without vitamin B₁₂, insignificant amounts of ethene were formed, while vitamin B₁₂ containing media converted 400 µM of 1,2-DCA within 3 days. Vitamin B₁₂ is a vitamin which is often required for bacterial growth. Also, the role of Vitamin B₁₂ in bacterial dechlorination has been described (e.g. Krasotkina et al. (2001) *J. Biol*. *Chem.* **276**,10991-40997; Adrian et al. (2000) *Biodegradation* **11,** 73-81). Propyliodide (50 µM) fully inhibited the dechlorination in the dark. The inhibition was light reversible as dechlorination starts in light conditions. Chloroform (50 µM), a known inhibitor of chlorinated ethene dehalogenases, only partially (<50%) inhibited the dechlorination of 1,2-DCA. Accumulating doses of 1,2-DCA accelerated the conversion rate. In media containing 0.1% (w/w) yeast extract, dechlorination rates by the pure culture of strain DCA1 exceeded 250 nmol Cl⁻ /mgₚᵣₒₜₑᵢₙ/min. The protein yield was measured as 0.8 gₚᵣₒₜₑᵢₙ /mol Cl⁻_{released}.

Besides 1,2-DCA, a defined range of other chlorinated alkanes sustained growth of strain DCA1, including 1,1,2-TCA; 1,2-D; 1,2-DCB; D-2,3-DCB, L-2,3-DCB and *meso*-2,3-DCB. Except for 1,1,2-TCA being selectively converted to VC, all other substrates were fully dechlorinated to the corresponding alkenes with at least 99.8% molar selectivity. In all cases, final concentrations dropped below 2 µM. [see table 2]

**Table 2:**

| ***Substrate range determination of the Desulfitobacterium of the present invention*** | | | |
|---|---|---|---|
| **Organic chlorosubstrate** | **Concentration** | **Growth** | **Product** |
| **Alkanes** | | | |
| Chloroethane | 400 µM | - | - |
| 1,2-DCA | 400 µM | + | ethene |
| 1,2-DBA | 400 µM | | ethene |
| 1,1,2-TCA | 400 µM | + | vinyl chloride |
| 1,1,1,2-TeCA | 400 µM | - | - |
| 1,1,2,2-TeCA | 400 µM | - | - |
| HCA | 400 µM | - | - |
| 1,2-dichloropropane | 400 µM | + | propene |
| 1,2-dibromopropane | 400 µM | + | propene |
| 1,2,3-trichloropropane | 400µM | - | - |
| 1,2-DCB | 400 µM | + | 1-butene |
| *meso*-1,2-DCB | 400 µM | + | *E*-but-2-ene |
| D-2,3-DCB | 400 µM | - | - |
| L-2,3-DCB | 400 µM | + | Z-but-2-ene |
| Ethenes | | | |
| PCE | 400 µM | - | - |
| 1,1,2-TCE | 400 µM | - | - |
| *cis*-DCE | 400µM | - | - |
| *trans*-DCE | 400 µM | - | - |
| VC | 400 µM | - | - |
| 3,4-dichlorobut-1-ene | 400 µM | - | - |
| **Aromatic substrates** | | | |
| 2,4,6-TCP | 100 µM | - | - |
| 2,4-DCP | 100 µM | - | - |
| 3-CI-4-hydroxyphenylacetate - | 600 µM | - | - |
| 1,3,4-TCB | 100 µM | - | - |
| **Other e**^{**-**}**-acceptors** | | | |
| meso-2,3-dibromosuccinate | 200 µM | - | - |
| 2,3-DC-propanol | 400 µM | - | - |
| *trans*-1,2-DC-cyclohexane | 200 µM | - | - |
| lindane | 100 µM | - | - |
| Abbreviatons: DBA: dibromoethane; TCA, trichloroethane; TeCA: tetrachloroalkane; HCA: hexachloroethane; DCB: dichlorobutane; PCE: tetrachloroethene: TCE: trichloroethene ; DCE : dichloroethene ; VC : vinylchloride: TCP: trichlorophenol; DCP: dichlorophenol. | | | |

Higher chlorinated alkanes such as hexa-, penta- and tetrachloroethanes were not dichloroeliminated. However, 1,1,2-trichloroethane still supported growth, producing vinylchloride, illustrating the exclusive dichloroelimination mechanism. Moreover, it indicates that the catalytic center of the dehalogenase is hindered, as soon as more than 3 chlorine substituents are present on both vicinal carbon atoms. Consistent with the exclusivity for dichloroeliminations, no chlorinated methanes, monochloroalkanes or non-vicinal dichloroalkanes were dechlorinated. When functional groups flanked the vicinal dichlorinated C2 core in the chlorinated substrate molecule, as in 2,3-dichloropropane (1 alcohol group), meso-2,3-dibromosuccinate (1 carboxylic acid group at both sides), 1,2,3-trichloropropane (1alkylchloride group) or lindane (2 alkylchloride groups at both sides), no dechlorination reaction was observed.

The dehalogenation mechanism of *Desulfitobacterium dichloroeliminans strain* DCA1 is catalyzed by a very specific enzyme. The dehalogenase of *Desulfitobacterium* strain DCA1 can convert 3 different 2,3-DCB stereoisomers. The *meso* stereoisomer can be selectively transformed to *trans*-2-butene, while both *dl*-enantiomers produce *cis*-2-butene. In the most stable staggered conformers of the *meso* and both *dl* compounds, both chlorine atoms are opposite to each other. Especially these conformers result in the production of *trans*-2-butene respectively *cis*-2-butene, only when a stereoselective *anti* dichloroelimination is carried out. The high stereoselectivity indicates that a non-radical mechanism is involved. Moreover, the less stable eclipsed *meso* and *dl* conformers, which are far less probable to appear in aqueous solution below 303 K, require a *syn* dichloroelimination on vicinal adjacent chlorine atoms. The latter reaction results in the opposite product formation, as eclipsed *meso* conformers would produce *trans*-2-butene while the lowest energy eclipsed *dl* conformers produce *cis*-2-butene. These products were not detected. Moreover, the high dechlorination rate, supports that the most probable conformer is taken as the substrate of the dehalogenase of strain DCA1. Hence, a selective two-electron *anti* dichloroelimination reaction is present.

In conclusion, phylogenetic, morphological and physiological characterization indicates that *Desulfitobacterium* strain DCA1 belongs to a new species of the *Desulfitobacterium* genus. Strain DCA1 combines a full dechlorination with an elevated selectivity and a high conversion rate. The strain bears a very specific dehalogenase enzyme catalyzing a selective one-step anti dichloroelimination process.

### EXAMPLE 3 Production of bacterial consortium comprising Desulfitobacterium dichloroeliminans strain DCA1

The present example describes the production of a bacterial consortium comprising *Desulfitobacterium dichloroeliminans* strain DCA1.

The nutrient solution for *Desulfitobacterium dichloroeliminans* strain *DCA1* was prepared by using (per liter) resazurin (1 ml 0.2%), a basal medium (20 ml) and water (979 ml). The basal medium consisted of MnCl₂.4H₂O (2.0 g/l), Na₂SO₄ (3.5 g/l), KH₂PO₄ (10.0 g/l), NH₄Cl (12.5 g/l), NaCI (50.0 g/l), MgCl₂.6H₂O (20.0 g/l), KCI (25.0 g/l) and CaCl₂.2H₂O (7.5 g/l). 0.5 g/l yeast extract and 40 mM pyruvate were added to the medium. Subsequently, 120 ml serum bottles were filled with 25 ml of the above-described medium and closed.

The medium was made anaerobic, using N₂ (or Ar) gas, the anaerobic medium was autoclaved and 4 mM 2,3-DCB (nominal concentration) was added. The substrate was redosed after about 5 to 6 days to enhance cell mass production when shaking on 28°C. In addition, 40 mM pyruvate was added as a fermentation substrate. 1 vol% of a bacterial culture of *Desulfitobacterium dichloroeliminans* strain DCA1 LMG P-21439 and a 1 vol% of a bacterial culture of *Enterococcus* sp. LMG 12901 was introduced in the medium.

Following the above-described steps, a bi-bacterial consortium comprising *Desulfitobacterium dichloroeliminans* strain DCA1 in a density exceeding 10⁸ bacteria /ml, and *Enterococcus* cells not exceeding 10⁸ bacteria/ml was obtained. A 1 vol% inoculation of this bi-bacterial consortium was able to dechlorinate 400 µM 1,2-DCA containing groundwater at 13°C within 10 days in the presence of a technical grade lactate. At 18°C, the rate almost doubled. In parallel tests without inoculum, ethene production could not be detected after several months.

In conclusion, a dense bacterial consortium comprising *Desulfitobacterium dichloroeliminans* strain DCA1 and an *Enterococcus* strain, which can effectively convert dichloroalkane substrates with vicinal chlorine atoms, can be easily prepared.

### EXAMPLE 4 Use of Desulfitobacterium dichloroeliminans strain DCA1 for in situ remediation of a contaminated site.

The bacterial consortium according to the invention can be used for *in situ* remediation of a site contaminated with di- or trichloroalkanes, having vicinal chlorine atoms, preferably under anaerobic conditions.

For example, 6 m³ of contaminated water can be treated with NaHCO₃ in order to achieve a pH between 7 and 8. After this pH adjustment, some 100 liter full grown bacterial culture (Optical Density > 0.3) containing >90% *Desulfitobacterium* strain DCA1 cells, can be injected. Degradation of the 1,2-DCA containing groundwater can be followed in function of time over a period of 10 days. Some 8 m³ of 1,2-DCA polluted groundwater at 17 m depth, can be inoculated with the new strain DCA1. Hydrogeological, bacterial and chemical follow-up can be arranged to monitor the biodegradation. The dehalogenating activity of the bacteria of the present invention has an optimum between 25 and 30 °C. The activity decreases with a factor of about 2 when the temperature is decreased with 10 °C. Temperatures of 10-15 °C are typical for groundwater. Acceptable levels of degradation are still achieved between 10-20 °C. Bacterial growth stops at temperatures of 35 °C and higher.

## Claims

1. A composition comprising bacteria of *Desulfitobacterium* sp. **characterised in** said composition being capable of dehalogenating a haloalkane under anaerobic conditions.

2. The composition according to claim 1 wherein said haloalkane is a dihaloalkane or trihaloalkane with vicinal halogen atoms.

3. The composition according to claim 2, wherein said dihaloalkane or trihaloalkane with vicinal halogen atoms is selected from the group consisting of 1,2-dichloroethane (1,2-DCA), 1,2-dichloropropane (1,2-D), 1,2-dichlorobutane (1,2-DCB), D-2,3-dichlorobutane (D-2,3-DCB), L-2,3-dichlorobutane (L-2,3-DCB), meso-2,3-dichlorobutane (meso-2,3-DCB), 1,1,2-trichloroethane (1,1,2-TCA), or mixtures thereof.

4. The composition according to any of claims 1 to 3 further comprising living Vitamin K secreting bacteria or further comprising a medium conditioned by said Vitamin K secreting bacteria, said vitamin K secreting bacteria preferably being an *Enterococcus sp*.

5. The composition according to any of claims 1 to 3 further comprising a Vitamin K. .

6. The composition according to claim 1 wherein dehalogenating of said haloalkane occurs at a rate of at least 50 nmol Cl⁻ per mg bacterial protein per minute or wherein dehalogenating said haloalkane occurs with a conversion efficiency of more than 50%

7. An isolated bacterium of the *Desulfitobacterium* genus **characterised in** having a 16S ribosomal DNA sequence as depicted in SEQ ID NO:1 or a 16S ribosomal DNA sequence being at least 97,0 % identical to SEQ ID NO:1.

8. The bacterium according to claim 7 wherein said bacterium is the *Desulfitobacterium dichloroeliminans* species, deposited under accession number LMG P-21439.

9. A growth medium for supporting the dehalogenation of haloalkanes by *Desulfitobacterium sp* **characterised in that** it comprises a Vitamin K.

10. A method for dehalogenating a haloalkane comprising the steps of:
- contacting an environment comprising said haloalkane with a composition according to any of claims 1 to 6 in a quantity effective to dehalogenate said haloalkane and
- ensuring conditions supporting metabolism, growth and reproduction of *Desulfitobacterium sp*. bacteria.

11. The method according to claim 10 wherein the haloalkane is a dihaloalkane or trihaloalkane with vicinal halogen atoms.

12. The method according to claim 11, wherein said dihaloalkane or trihaloalkane with vicinal halogen atoms is selected from the group consisting of 1,2-dichloroethane (1,2-DCA), 1,2-dichloropropane (1,2-D), 1,2-dichlorobutane (1,2-DCB), D-2,3-dichlorobutane (D-2,3-DCB), L-2,3-dichlorobutane (L-2,3-DCB), meso-2,3-dichlorobutane (meso-2,3-DCB) and 1,1,2-trichloroethane (1,1,2-TCA).

13. The method according to claim 10 wherein said environment is a contaminated site, and wherein the step of contacting said environment with said composition occurs in situ.

14. The method according to claim 10 wherein said step of ensuring conditions supporting metabolism, growth and reproduction comprises:
- maintaining or providing a pH value between 6 and 8.
- maintaining or providing a temperature between 10 °C and 30 °C
- maintaining or providing anaerobic conditions.

15. A method for preparing a growth medium according to claim 9 comprising the steps of:
- introducing a nutrient solution in an incubator vessel,
- making anaerobic conditions,
- sterilising said nutrient solution,
- introducing a bacterial culture of Vitamin K secreting bacteria, preferably *Enterococcus sp*,.
- maintaining conditions suitable to allow growth and proliferation of the bacteria, and
- optionally cooling said vessel and contents to a temperature which prevents further growth of bacteria and is suitable for storage of the culture.

16. Use of a composition according to any of claims 1 to 6 or of a growth medium according to claim 9 in dehalogenating a haloalkane, preferably under anaerobic conditions.

17. Use according to claim 16, wherein the dehalogenation is the remediation in situ of a contaminated site.

18. Use according to claim 16 or 17 wherein said haloalkane is a dihaloalkane or trihaloalkane with vicinal halogen atoms.

19. Use according to claim 18, wherein said dihaloalkane or trihaloalkane with vicinal halogen is selected from the group consisting of 1,2-dichloroethane (1,2-DCA), 1,2-dichloropropane (1,2-D), 1,2-dichlorobutane (1,2-DCB), D-2,3-dichlorobutane (D-2,3-DCB), L-2,3-dichlorobutane (L-2,3-DCB), meso-2,3-dichlorobutane (meso-2,3-DCB) and 1,1,2-trichloroethane (1,1,2-TCA).

20. A composition comprising bacteria **characterised in** said bacteria being capable of dehalorespiring a haloalkane with vicinal halogen atoms under anaerobic conditions.


SEQUENCE LISTING

<110> Universiteit Gent

<120> method for anaerobic bacterial degradation of haloalkanes and novel ba
cterial
species therefor

<130> R 2335 EP

<150> EP02447061.9
<151> 2002-04-10

<160> 1

<170> PatentIn version 3.1

<210> 1
<211> 1467
<212> DNA
<213> Desulfitobacterium dichloroeliminans

<220>
<221> gene
<222> (1)..(1467)
<223> 16S ribosomal DNA

<400> 1
cactgagtgc tagacgttag ttagtggcgg acgggtgagt aacgcgtgga taacctaccc 60

attagaccgg gacaaccctt ggaaacgagg gctaataccg gataagaata tttcgcggca 120

tcgcgagata aggaaagatg gcctctgart atgctatcgt taatggatgg atccgcgtct 180

gattagctag ttggtggggt aaaggcctac caaggcgacg atcagtagcc ggcctgagag 240

ggtgaacggc cacactggga ctgagacacg gcccagactc ctacgggagg cagcagtggg 300

gaatcttccg caatggacga aagtctgacg gagcaacgcc gcgtgtacga cgaaggcctt 360

cgggttgtaa agtactgtct tcagggacga acggtattta tgtaaataat gtagatacat 420

gacggtacct gaggaggaag ccccggctaa ctacgtgcca gcagccgcgg taatacgtag 480

ggggcaagcg ttgtccggaa tcattgggcg taaagggcgc gtaggcggat aattaagtct 540

ggtgtgaaaa cctagggctc aaccctggga ctgcatcgga aactggttat cttgaggaca 600

ggagaggaaa gtggaattcc acgtgtagcg gtgaaatgcg tagatatgtg gaggaacacc 660

agtggcgaag gcgactttct ggactgtaac tgacgctgag gcgcgaaagc gtggggagca 720

aacaggatta gataccctgg tagtccacgc cgtaaacgat gagtgctagg tgtagagggt 780

atcgacccct tctgtgccgc agttaacaca ataagcactc cgcctgggga gtacggccgc 840

aaggttgaaa ctcaaaggaa ttgacggggg cccgcacaag cggtggatta tgtggtttaa 900

ttcgacgcaa cgcgaagaac cttaccaagg cttgacatcc tacgaatcct gaggaaactt 960

aggagtgccc ttcggggagc gtagagacag gtggtgcatg gttgtcgtca gctcgtgtcg 1020

tgagatgttg ggttaagtcc cgcaacgagc gcaaccccta tgtttagttg ctaacgagta 1080

aggtcgagca ctctagacag actgccggtg acaaaccgga ggaaggtggg gatgacgtca 1140

aatcatcatg ccccttatgt cttgggctac acacgtaata caatggccgg tacagacgga 1200

agcgaagccg tgaggtgaag cgaatccgag aaagccggtc tcagttcgga ttgttctctg 1260

caactcgaga acatgaagtc ggaatcgcta gtaatcgcag gtcagcatac tgcggtgaat 1320

acgttcccgg gccttgtaca caccgcccgt cacaccacga aagtctgcaa cacccgaagc 1380

cggtgaggta acccgaaagg gagctagccg tcgaaggtgg ggccgatgat tggggtgaag 1440

tcgtaacaag gtagccgtat cggaagg 1467